# EUROPÄISCHE PATENTSCHRIFT

(11) **EP 1 709 909 B1**
(45) Veröffentlichungstag und Bekanntmachung des Hinweises auf die Patenterteilung: **19.08.2009**
(21) Anmeldenummer: 06007203.0
(22) Anmeldetag: 05.04.2006
(51) Int. Cl.: A61B 17/04

(54) **Vorrichtung zum Zuführen von chirurgischem Nahtmaterial zu einer Nadel**
Device for feeding a surgical thread to a needle
Dispositif d'alimentation des sutures chirurgicales à une aiguille

(30) Priorität: 06.04.2005 DE 102005015687
(43) Veröffentlichungstag der Anmeldung: 11.10.2006
(73) Patentinhaber: Karl Storz GmbH & Co. KG, 78532 Tuttlingen (DE)
(72) Erfinder: Oberländer, Martin, 78234 Engen (DE); Sauer, Michael, 78532 Tuttlingen (DE)
(74) Vertreter: Hofmeister, Frank

(56) Entgegenhaltungen:
- DE-A1- 2 257 728
- US-A- 919 138
- US-A- 962 218
- US-A- 1 180 975
- US-A- 2 474 463
- US-A- 2 808 055
- US-A- 3 186 262
- US-A- 4 012 010
- US-A- 5 776 151

## Beschreibung

Die Erfindung betrifft eine Vorrichtung zum Zuführen von chirurgischem Nahtmaterial zu einer Nadel, mit einem Schaft, an dessen distalem Ende die Nadel festlegbar ist und dessen proximales Ende als mit einem offenen Führungskanal für das zuzuführende Nahtmaterial versehener Griffteil ausgebildet ist und der Führungskanal zumindest abschnittweise mechanisch über mindestens eine das Nahtmaterial im Führungskanal zurückhaltende Verschlussvorrichtung verschließbar ist.

Derartige Vorrichtungen dienen dazu, das chirurgische Nahtmaterial ausgehend von einer Garnspule einer in der Regel zumindest teilweise als Hohlnadel ausgebildeten chirurgischen Nadel zuzuführen.

Eine gattungsgemäße Vorrichtung ist aus der US 1 180 975 bekannt. Bei dieser bekannten Vorrichtung ist es erforderlich, das Nahtmaterial um die Verschlussvorrichtung zu wickeln, um ein seitliches Austreten des Nahtmaterials aus dem Führungskanal am Kopf der Verschlussvorrichtung zu verhindern. Dieses Umwickeln der Verschlussvorrichtung mit dem Nahtmaterial behindert jedoch die freie Fadenführung des Nahtmaterials beim Nachziehen des Nahtmaterials.

Weiterhin ist aus der US-Patentschrift 2 808 055 ist eine Vorrichtung zum Zuführen von chirurgischem Nahtmaterial bekannt, die aus einem Griffteil und einer im Griffteil angeordneten Transportvorrichtung besteht, über die das Nahtmaterial durch Vor- und Zurückschieben eines Gleitschuhs der Nadel zuführbar ist. Nachteilig an dieser bekannten Zuführvorrichtung ist, dass der konstruktive Aufbau der Transportvorrichtung sehr groß ist und darüber hinaus die Bedienung der Transportvorrichtung einiger Übung bedarf.

Eine weitere einfacher aufgebaute Vorrichtung zum Zuführen von chirurgischem Nahtmaterial zu einer Nadel, die keine separate Transportvorrichtung aufweist, ist aus der Praxis bekannt. Diese bekannten Vorrichtung besteht im Wesentlichen aus einem Schaft, an dessen distalem Ende die Nadel festlegbar ist und dessen proximales Ende als mit einem offenen Führungskanal für das zuzuführende Nahtmaterial versehener Griffteil ausgebildet ist. Das Zuführen des in dem Führungskanal gelagerten Nahtmaterials hin zur Nadel erfolgt durch Nachschieben von weiterem Nahtmaterial über das proximale Ende des Schaftes in den Führungskanal. Nachteilig bei dieser ansonsten sehr zuverlässigen Vorrichtung ist, dass das zur Nadel nach vorne geschobene Nahtmaterial aufgrund seiner Eigensteifigkeit dazu neigt sich aufzubäumen und nach oben aus dem Führungskanal herauszutreten, sobald im vorderen Bereich des Nahtmaterials, beispielsweise beim Einführen in die Nadel, ein geringfügig größerer Widerstand auftritt. Sobald aber das Nahtmaterial aus dem Führungskanal heraustritt, ist ein weiteres Vorschieben des Nahtmaterials hin zur Nadel unmöglich.

Davon ausgehend liegt der Erfindung die **Augabe** zugrunde, eine Vorrichtung zum Zuführen von chirurgischem Nahtmaterial zu einer Nadel zu schaffen, die bei einfachem Aufbau einen zuverlässigen Transport des Nahtmaterials zur Nadel gewährleistet.

Die **Lösung** dieser Aufgabenstellung ist gemäß einer ersten Ausführungsform erfindungsgemäß dadurch gekennzeichnet, dass jede Verschlussvorrichtung zwei aneinander anliegende Sperrelemente aus einem elastischen Material aufweist, so dass das Nahtmaterial von oben zwischen den aneinander anliegenden Sperrelementen hindurch in den Führungskanal eindrückbar ist.

Durch den erfindungsgemäßen Vorschlag, den Führungskanal zumindest abschnittweise mechanisch über mindestens eine Verschlussvorrichtung verschließbar so auszugestalten, dass das Nahtmaterial zwischen den aneinander anliegenden Sperrelementen hindurch von oben in den Führungskanal eindrückbar ist, wird das Aufbäumen und Heraustreten des Nahtmaterials aus dem Führungskanalverhindert.

Aufgrund der Eigensteifigkeit des Nahtmaterials ist es ausreichend, den Führungskanal nur abschnittweise zu verschließen. Der verbleibende Zwischenraum zwischen zwei verschlossenen Bereichen des Kanals kann so gewählt werden, dass ein erneutes Aufbäumen des Nahtmaterials verhindert wird.

Gemäß einer ersten erfindungsgemäßen Ausführungsform wird vorgeschlagen, dass zum Verschließen des Führungskanals im Führungskanal mindestens ein das Nahtmaterial im Führungskanal zurückhaltendes Verschlusselement angeordnet ist.

Mit einer bevorzugten Ausführungsform der Erfindung wird vorgeschlagen, dass das mindestens eine Verschlusselement mindestens ein den Führungskanal oberhalb des Nahtmaterials zumindest teilweise verschließendes Sperrelement aufweist. Dieses oberhalb des Nahtmaterials angeordnete Sperrelement blockiert ein mögliches Aufbäumen des Nahtmaterials und ermöglicht so das weitere Vor- und Zurückschieben des Nahtmaterials entlang dem Führungskanal.

Zur Ausbildung der Sperrelemente wird erfindungsgemäß vorgeschlagen, dass diese vorzugsweise als aus einem Kunststoff- oder Gummi-Material bestehende O-Ringe ausgebildet sind. Die Ausbildung der Sperrelemente als zwei aneinander anliegende O-Ringe aus einem elastischen Material, wie beispielsweise einem Kunststoff- oder Gummi-Material, hat den Vorteil, dass das Nahtmaterial von oben zwischen den aneinander anliegenden Sperrelementen hindurch in den Führungskanal drückbar ist, ohne das Verschlusselement und/oder das Sperrelement in einem gesonderten Arbeitsschritt öffnen zu müssen.

Weiterhin wird mit der Erfindung vorgeschlagen, dass jedes Verschlusselement als von der dem Führungskanal gegenüberliegenden Seite in den Griffteil einschraubbarer Gewindebolzen ausgebildet ist, in dessen Schaft eine dem Führungskanal entsprechende Nut ausgebildet ist, wobei erfindungsgemäß die Sperrelemente an den freien Enden des durch die Nut zweigeteilten Schafts angeordnet sind.

Um zu gewährleisten, dass die in dem Schaft des Schraubbolzens ausgebildete Nut immer fluchtend mit dem im Griffteil der Vorrichtung ausgebildeten Führungskanal ausrichtbar ist, ist der die Nut aufweisende Schaft frei drehbar an dem Gewindebolzen gelagert.

Das lagerichtige Einsetzen des Gewindebolzens in den Griffteil kann weiterhin dadurch vereinfacht werden, dass am drehbaren Schaft mindestens ein Positionierelement ausgebildet ist, das mit einem korrespondierenden Positionierelement im Griffteil zusammenwirkt. Vorteilhafterweise ist das am drehbaren Schaft ausgebildete Positionierelement als Längsrippe ausgebildet ist, die in eine entsprechende Längsnut im Griffteil einsetzbar ist, so dass immer eine fluchtende Ausrichtung von Nut und Führungskanal gewährleistet ist.

Die **Lösung** der Aufgabenstellung ist gemäß einer zweiten Ausführungsform erfindungsgemäß dadurch gekennzeichnet, dass die mindestens eine Verschlussvorrichtung eine am Griffteil festlegbare und das Griffteil umgreifende Klammer ist, die als Federelement so ausgebildet ist, dass das Nahtmaterial von oben durch eine Öffnung im Federelement hindurch in den Führungskanal eindrückbar ist.

Durch die Ausbildung der Klammer als Federelement lässt sich die Klammer einfach auf den Griffteil aufsetzen und das Nahtmaterial durch eine im Federelement angeordnete Öffnung in den Führungskanal einzuführen.

Weiterhin wird mit der Erfindung vorgeschlagen, dass am distalen Ende des Griffteils im Schaft eine Griffmulde zum klemmenden Ergreifen des Nahtmaterials ausgebildet ist. Die Griffmulde ist dabei so ausgebildet, dass diese an die Haltung und Form des Daumen der die Vorrichtung haltenden Hand angepasst ist. Das zeitweise klemmende Halten des Nahtmaterials ist vorteilhaft, um ein unbeabsichtigtes Herausziehen des Nahtmaterials aus der Nadel zu verhindern.

Schließlich wird mit der Erfindung vorgeschlagen, dass am Boden der Griffmulde eine Führungsnut zur Aufnahme des Nahtmaterials ausgebildet ist, um einerseits auch beim klemmenden Ergreifen des Nahtmaterials dieses in der richtigen Ausrichtung zur Nadel zu halten und andererseits ein Knicken des Nahtmaterials zu verhindern.

Weitere Merkmale und Vorteile der Erfindung ergeben sich anhand der zugehörigen Zeichnung, in der zwei Ausführungsbeispiele einer erfindungsgemäßen Vorrichtung zum Zuführen von chirurgischem Nahtmaterial zu einer Nadel nur beispielhaft dargestellt sind. In der Zeichnung zeigt:
- Fig. 1: eine teilweise geschnittene Seitenansicht einer ersten Ausführungsform einer erfindungsgemäßen Vorrichtung;
- Fig. 2: einen perspektivischen Längsschnitt durch die Vorrichtung gemäß Fig. 1;
- Fig. 3: einen vergrößerten Schnitt entlang der Linie III-III gemäß Fig. 1;
- Fig. 4: eine vergrößerte perspektivische Ansicht des Details IV gemäß Fig. 2 und
- Fig. 5: eine zweite Ausführungsform einer erfindungsgemäßen Vorrichtung im Querschnitt.

Die in den Abbildungen dargestellte Vorrichtung zum Zuführen von chirurgischem Nahtmaterial zu einer Nadel besteht im Wesentlichen aus einem Schaft 1 an dessen distalem Ende über ein Adapterstück 2 eine nicht dargestellte chirurgische Nadel, insbesondere eine mit einer Durchgangsbohrung versehene Hohlnadel, festlegbar ist. Das proximale Ende des Schaftes 1 ist als Griffteil 3 ausgeformt, in dem eine in Längsrichtung des Schaftes 1 verlaufende Führungskanal 4 zur führenden Aufnahme des Nahtmaterials 5 ausgebildet ist.

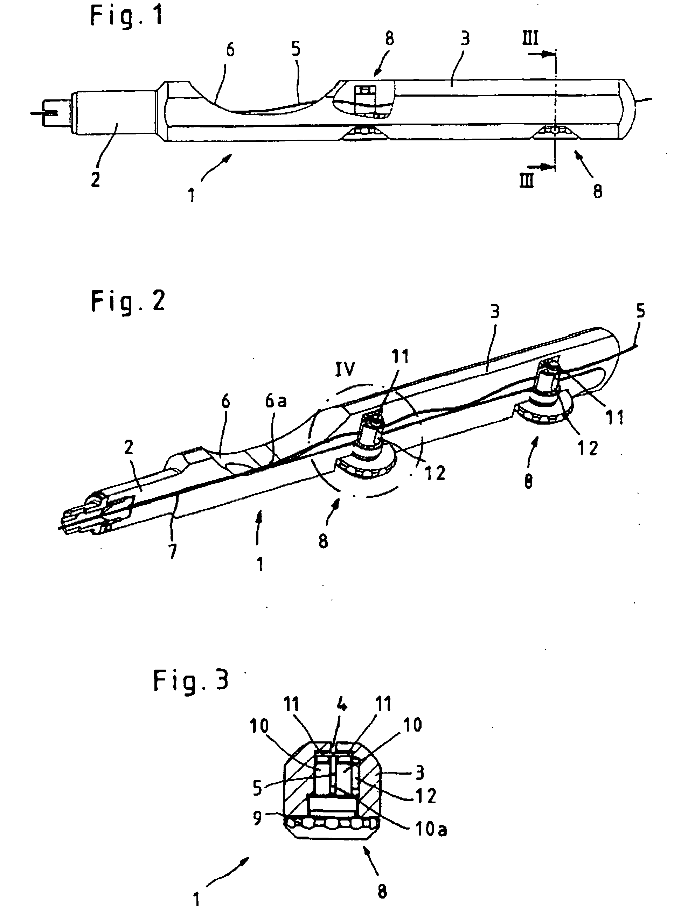

Wie weiterhin aus Fig. 1 und 2 ersichtlich, ist am distalen Ende des Griffteils 3, also im Bereich des Schaftes 1 zwischen dem Griffteil 3 und dem Adapterstück 2 eine Griffmulde 6 ausgebildet, die nach Lage und Form daran angepasst ist, dass sie zur Aufnahme des Daumens der den Schaft 1 haltenden Hand dient.

Um zu verhindern, dass das im Führungskanal 4 angeordnete und über eine Durchgangsbohrung 7 im Adapterstück 2 der Nadel zuzuführende Nahmaterial 5 beim Nachschieben des Nahtmaterials 5 über das proximale Ende des Schaftes 1 aus dem Führungskanal 4 heraustreten kann und sich aufgrund der Eigensteifigkeit des Nahtmaterials 5 aus dem Führungskanal 4 heraus aufbäumt, sobald distalseitig ein gewisser Widerstand, beispielsweise beim Einfädeln in die Nadel, auftritt, sind mechanische Mittel vorgesehen, um den Führungskanal 4 zumindest abschnittweise verschließen zu können.

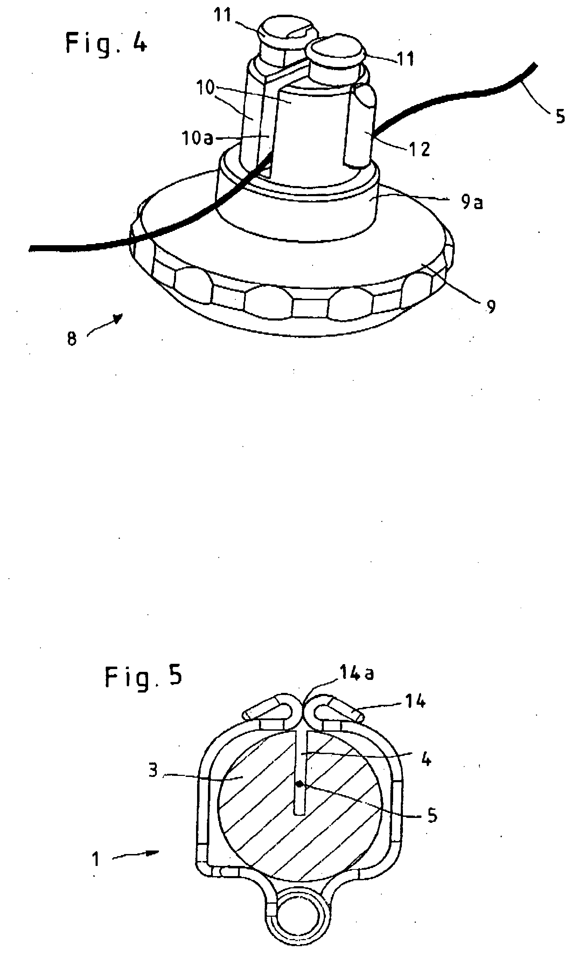

Bei der in den Abbildungen Fig. 1 bis 4 dargestellten ersten Ausführungsform erfolgt dieses Verschließen des Führungskanals 4 über zwei im Führungskanal 4 angeordnete Verschlusselemente 8, über die das Nahtmaterial 5 im Führungskanal 4 haltbar ist. Wie insbesondere aus den Abbildungen Fig. 2 und 4 ersichtlich, sind die Verschlusselemente 8 als von der der Öffnung des Führungskanals 4 gegenüberliegenden Seite in den Griffteil 3 des Schaftes 1 einschraubbare Gewindebolzen ausgebildet. Der das Verschlusselement 8 bildende Gewindebolzen besteht dabei im Wesentlichen aus einem mit einem Außengewinde 9a versehenen Grundkörper 9 sowie einem frei drehbar am Grundkörper 9 gelagerten Schaft 10, der durch eine dem Führungskanal 4 entsprechende senkrechte Nut 10a in zwei Schaftschenkel unterteilt ist.

Das eigentliche Zurückhalten des Nahtmaterials 5 im Führungskanal 4 bzw. in der Nut 10a des Schaftes 10 erfolgt über an den freien Enden der Schaftschenkel angeordnete Sperrelemente 11, die bei der dargestellten Ausführungsform als zwei aneinander anliegenden O-Ringe ausgebildet sind, die aus einem Kunststoff- oder Gummi-Material bestehen. Wie insbesondere die Schnittdarstellung gemäß Fig. 3 zeigt, verschließen die beiden aneinander anliegenden O-Ring Sperrelemente 11 die im Schaft 10 des Verschlusselements 8 ausgebildete Nut 10a und somit auch den Führungskanal 4 derart, dass das im Führungskanal 4 und der Nut 10a angeordnete Nahtmaterial nicht durch bloßes Aufbäumen nach oben aus dem Führungskanal 4 heraustreten kann.

Um das lagerichtige Einsetzen der Verschlusselemente 8 in den Griffteil 3 des Schaftes 1 zu erleichtern, bei dem die Nut 10a mit dem Führungskanal 4 fluchtet, ist am Schaft 10 neben der drehbaren Lagerung des Schaftes 10 am Grundkörper 9 ein Positionierungselement 12 ausgebildet, das bei der dargestellten Ausführungsform als Längsrippe ausgeformt mit einer entsprechenden, nicht dargestellten Längsnut im Griffteil 3 zusammenwirkt.

Bei der in Abbildung Fig. 5 dargestellten Ausführungsform erfolgt das mechanische Verschließen des Führungskanals 4 dadurch, dass der im Griffteil 3 ausgebildete nach oben offene Führungskanal 4 mittels einer Verschlussvorrichtung zumindest abschnittweise verschließbar ist.

Bei der in Fig. 5 dargestellten zweiten Ausführungsform erfolgt das Verschließen des Führungskanals 4 über eine am Griffteil 3 festlegbare und diesen umgreifende Klammer 14, die wie dargestellt als Federelement ausgebildet sein kann. Alternativ zu der Ausbildung als Klammer 14 ist es auch möglich, die Abdeckung als im Wesentlichen formschlüssig auf den Griffteil 3 aufschiebbare Hülse auszugestalten. Die Klammer 14 bzw. die Hülse werden nach dem Einlegen des Nahtmaterials 5 in den Führungskanal 4 auf den Griffteil 3 des Schaftes 1 aufgesetzt, um den Führungskanal 4 oberflächlich zu verschließen und das versehentliche Austreten des Nahtmaterials 5 aus dem Führungskanal 4 zu verhindern. Im Falle der Klammer 14 ist es jedoch auch möglich, diese bereits vor dem Einlegen des Nahtmaterials 5 in den Führungskanal 4 auf das Griffteil 3 des Schaftes 1 aufzusetzen und das Nahtmaterial 5 durch eine im Wesentlichen fluchtend mit dem Führungskanal 4 angeordnete Öffnung 14a der Klammer 14 in den Führungskanal 4 einzuführen.

Die Benutzung der Vorrichtung zum Zuführen von chirurgischem Nahtmaterial 5 zu einer Nadel wird nachfolgend anhand der in den Abbildungen Fig. 1 bis 4 dargestellten Ausführungsform beschrieben:

Zu Beginn wird die mit dem Nahtmaterial 5 zu bestückende chirurgische Nadel am Adapterstück 2 des Schaftes 1 der Vorrichtung festgelegt.

Anschließen wird das Nahtmaterial 5 von oben in den nach oben offenen Führungskanal 4 eingelegt, bis dieses auf den Sperrelemente 11 der beiden im Führungskanal 4 angeordneten Verschlusselemente 8 aufliegt. Da die beiden aneinander anliegenden, als O-Ringe ausgebildeten Sperrelemente 11 aus einem elastischen Kunststoff- oder Gummi-Material bestehen, ist es möglich, das Nahtmaterial 5 von oben durch die beiden aneinander anliegenden Sperrelemente 11 hindurch zu drücken, so dass das Nahtmaterial 5 im Bereich der Verschlusselemente 8 innerhalb der Nuten 10a zu liegen kommt.

Nachfolgend wird das Nahtmaterial 5 in die Durchgangsbohrung 7 eingefädelt und durch Nachschieben des Nahtmaterials 5 über das proximale Ende des Schaftes 1 das Nahtmaterial 5 in distaler Richtung nach vorne geschoben, bis es wieder aus der am Adapterstück 2 festgelegten Nadel austritt. Selbstverständlich ist es auch möglich, das Einfädeln des Nahtmaterials 5 in die Durchgangsbohrung 7 vorher auszuführen, das heißt, bevor das Nahtmaterial 5 durch die Sperrelemente 11 gedrückt wird.

Durch die Sperrelemente 11 der Verschlusselemente 8 wird verhindert, dass sich das Nahtmaterial 5 aufbäumt und aus dem Führungskanal 4 heraustritt, sobald beim Vorschieben des Nahtmaterials distalseitig ein Widerstand auftritt. Zum Einlegen des Natmaterials 5 in den Führungskanal 4 und die Nuten 10a lässt sich das Nahtmaterial 5 aufgrund der Flexibilität des Materials der Sperrelemente 11 zwischen den Sperrelementen 11 hindurch pressen, jedoch verhindern diese Sperrelemente 11 das Heraustreten des Nahtmaterials 5, da die im normalen Gebrauch beim Vorschieben des Nahtmaterials 5 auftretende Druckkraft nicht so groß ist, dass dadurch die Klemmkraft der Sperrelemente 11 überwunden werden kann.

Die im Bereich zwischen dem Adapterstück 2 und dem Griffteil 3 im Schaft 1 ausgebildete Griffmulde 6 dient dazu, während der Betätigung der Vorrichtung das Nahtmaterial 5 klemmend Ergreifen zu können, um ein versehentliches Herausziehen des Nahtmaterials 5 aus der Nadel zu verhindern. Um einerseits auch beim klemmenden Ergreifen des Nahtmaterials das Nahtmaterial 5 in der richtigen Ausrichtung zur Nadel zu halten und andererseits ein Knicken des Nahtmaterials 5 zu verhindern, ist am Boden der Griffmulde 6 eine Führungsnut 6a ausgebildet.

Die solchermaßen ausgebildete Vorrichtung zum Zuführen von chirurgischem Nahtmaterial 5 zu einer Nadel zeichnet sich dadurch aus, dass sie bei einfachem konstruktivem Aufbau und einfacher Handhabung einen zuverlässigen Transport des Nahtmaterials 5 zur Nadel gewährleistet.

### Bezugszeichenliste

- 1: Schaft
- 2: Adapterstück
- 3: Griffteil
- 4: Führungskanal
- 5: Nahtmaterial
- 6: Griffmulde
- 6a: Führungsnut
- 7: Durchgangsbohrung
- 8: Verschlusselement
- 9: Grundkörper
- 9a: Außengewinde
- 10: Schaft
- 10a: Nut
- 11: Sperrelement
- 12: Positionierungselement

- 14: Klammer

- 14a: Öffnung

## Patentansprüche

1. Vorrichtung zum Zuführen von chirurgischem Nahtmaterial zu einer Nadel, mit einem Schaft (1), an dessen distalem Ende die Nadel festlegbar ist und dessen proximales Ende als mit einem offenen Führungskanal (4) für das zuzuführende Nahtmaterial (5) versehener Griffteil (3) ausgebildet ist und der Führungskanal (4) zumindest abschnittweise mechanisch über mindestens eine das Nahtmaterial (5) im Führungskanal (4) zurückhaltende Verschlussvorrichtung verschließbar ist,
**dadurch gekennzeichnet,**
**dass** jede Verschlussvorrichtung zwei aneinander anliegende Sperrelemente (11) aus einem elastischen Material aufweist, so dass das Nahtmaterial (5) von oben zwischen den aneinander anliegenden Sperrelementen (11) hindurch in den Führungskanal (4) eindrückbar ist.

2. Vorrichtung nach Anspruch 1, **dadurch gekennzeichnet, dass** die Verschlussvorrichtung als im Führungskanal (4) angeordnetes Verschlusselement (8) ausgebildet ist.

3. Vorrichtung nach Anspruch 2, **dadurch gekennzeichnet, dass** das mindestens eine Verschlusselement (8) mindestens ein den Führungskanal (4) oberhalb des Nahtmaterials (5) zumindest teilweise verschließendes Sperrelement (11) aufweist.

4. Vorrichtung nach Anspruch 3, **dadurch gekennzeichnet, dass** das mindestens eine Sperrelement (11) als aus einem Kunststoff- oder Gummi-Material bestehender O-Ring ausgebildet ist.

5. Vorrichtung nach einem der Ansprüche 2 bis 4, **dadurch gekennzeichnet, dass** jedes Verschlusselement (8) als von der dem Führungskanal (4) gegenüberliegenden Seite in den Griffteil (3) einschraubbarer Gewindebolzen ausgebildet ist, in dessen Schaft (10) eine dem Führungskanal (4) entsprechende Nut (10a) ausgebildet ist.

6. Vorrichtung nach Anspruch 5, **dadurch gekennzeichnet, dass** der die Nut (10a) aufweisende Schaft (10) frei drehbar an dem Gewindebolzen gelagert ist.

7. Vorrichtung nach Anspruch 5 oder 6, **dadurch gekennzeichnet, dass** die Sperrelemente (11) an den freien Enden des durch die Nut (10a) zweigeteilten Schafts (10) angeordnet sind.

8. Vorrichtung nach Anspruch 6 oder 7, **dadurch gekennzeichnet, dass** am drehbaren Schaft (10) mindestens ein Positionierelement (12) ausgebildet ist, das mit einem korrespondierenden Positionierelement im Griffteil (3) zusammenwirkt.

9. Vorrichtung nach Anspruch 8, **dadurch gekennzeichnet, dass** am drehbaren Schaft (10) ausgebildete Positionierelement (12) als Längsrippe ausgebildet ist, die in eine entsprechende Längsnut im Griffteil (3) einsetzbar ist.

10. Vorrichtung zum Zuführen von chirurgischem Nahtmaterial zu einer Nadel, mit einem Schaft (1), an dessen distalem Ende die Nadel festlegbar ist und dessen proximales Ende als mit einem offenen Führungskanal (4) für das zuzuführende Nahtmaterial (5) versehener Griffteil (3) ausgebildet ist und der Führungskanal (4) zumindest abschnittweise mechanisch über mindestens eine das Nahtmaterial (5) im Führungskanal (4) zurückhaltende Verschlussvorrichtung verschließbar ist,
**dadurch gekennzeichnet,**
**dass** die mindestens eine Verschlussvorrichtung eine am Griffteil (3) festlegbare und das Griffteil (3) umgreifende Klammer (14) ist, die als Federelement so ausgebildet ist, dass das Nahtmaterial (5) von oben durch eine Öffnung (14a) im Federelement hindurch in den Führungskanal (4) eindrückbar ist.

11. Vorrichtung nach einem der Ansprüche 1 bis 10, **dadurch gekennzeichnet, dass** am distalen Ende des Griffteils (3) im Schaft (1) eine Griffmulde (6) zum klemmenden Ergreifen des Nahtmaterials (5) ausgebildet ist.

12. Vorrichtung nach Anspruch 11, **dadurch gekennzeichnet, dass** am Boden der Griffmulde (6) eine Führungsnut (6a) zur Aufnahme des Nahtmaterials (5) ausgebildet ist.

## Claims

1. Device for feeding surgical suture material to a needle, with a shaft (1) at whose distal end the needle can be secured and whose proximal end is designed as a grip part (3) provided with an open guide channel (4) for the feedable suture material (5), which guide channel (4) can be mechanically closed at least in some sections by at least one closure device that retains the suture material (5) in the guide channel (4), **characterized in that** each closure device has two adjoining blocking elements (11) made of an elastic material, such that the suture material (5) can be pressed into the guide channel (4) from above between the adjoining blocking elements (11).

2. Device according to Claim 1, **characterized in that** the closure device is designed as a closure element (8) arranged in the guide channel (4).

3. Device according to Claim 2, **characterized in that** the at least one closure element (8) has at least one blocking element (11) that at least partially closes the guide channel (4) above the suture material (5).

4. Device according to Claim 3, **characterized in that** the at least one blocking element (11) is designed as an O-ring made of a plastic or rubber material.

5. Device according to one of Claims 2 to 4,
**characterized in that** each closure element (8) is designed as a threaded bolt which can be screwed into the grip part (3) from the side opposite the guide channel (4) and in whose shaft (10) there is a groove (10a) that corresponds to the guide channel (4).

6. Device according to Claim 5, **characterized in that** the shaft (10) comprising the groove (10a) is mounted so as to be freely rotatable on the threaded bolt.

7. Device according to Claim 5 or 6, **characterized in that** the blocking elements (11) are arranged on the free ends of the shaft (10) divided in two by the groove (10a).

8. Device according to Claim 6 or 7, **characterized in that** at least one positioning element (12) is formed on the rotatable shaft (10) and interacts with a corresponding positioning element in the grip part (3).

9. Device according to Claim 8, **characterized in that** the positioning element (12) formed on the rotatable shaft (10) is designed as a longitudinal rib, which can be fitted into a corresponding longitudinal groove in the grip part (3).

10. Device for feeding surgical suture material to a needle, with a shaft (1) at whose distal end the needle can be secured and whose proximal end is designed as a grip part (3) provided with an open guide channel (4) for the feedable suture material (5), which guide channel (4) can be mechanically closed at least in some sections by at least one closure device that retains the suture material (5) in the guide channel (4), **characterized in that** the at least one closure device is a clip (14) which can be secured on the grip part (3) and which engages around the grip part (3) and is configured as a spring element, such that the suture material (5) can be pressed into the guide channel (4) from above through an opening (14a) in the spring element.

11. Device according to one of Claims 1 to 10,
**characterized in that** a recessed grip (6) is formed in the shaft (1) , at the distal end of the grip part (3), to allow the suture material (5) to be clamped tight.

12. Device according to Claim 11, **characterized in that** a guide groove (6a) for receiving the suture material (5) is formed on the floor of the recessed grip (6).

## Revendications

1. Dispositif pour l'amenée de matériau de suture chirurgical à une aiguille, comprenant un corps allongé (1) à l'extrémité distale duquel peut être fixée l'aiguille, et dont l'extrémité proximale est conçue sous forme de poignée (3) munie d'un canal de guidage (4) ouvert pour le matériau de suture à amener, le canal de guidage (4) pouvant être fermé mécaniquement, au moins par secteurs, par l'intermédiaire d'au moins un dispositif de fermeture retenant le matériau de suture (5) dans le canal de guidage (4),
**caractérisé en ce que** chaque dispositif de fermeture présente deux éléments de blocage (11) en un matériau élastique et en appui l'un contre l'autre, de manière telle que le matériau de suture (5) puisse être enfoncé dans le canal de guidage (4) par le haut, entre les éléments de blocage (11) en appui l'un contre l'autre.

2. Dispositif selon la revendication 1, **caractérisé en ce que** le dispositif de fermeture est réalisé en tant qu'élément de fermeture (8) agencé dans le canal de guidage (4).

3. Dispositif selon la revendication 2, **caractérisé en ce que** ledit au moins un élément de fermeture (8) présente au moins un élément de blocage (11) fermant au moins partiellement le canal de guidage (4) au-dessus du matériau de suture (5).

4. Dispositif selon la revendication 3, **caractérisé en ce que** ledit au moins un élément de blocage (11) est conçu sous forme de joint torique réalisé en un matériau du type matière plastique ou caoutchouc.

5. Dispositif selon l'une des revendications 2 à 4,
**caractérisé en ce que** chaque élément de fermeture (8) est réalisé sous forme de boulon fileté qui peut être vissé dans la poignée (3) à partir du côté opposé à celui où se trouve le canal de guidage (4), et dans la tige (10) duquel est formée une rainure (10a) correspondant au canal de guidage (4).

6. Dispositif selon la revendication 5, **caractérisé en ce que** la tige (10) présentant la rainure (10a) est montée librement rotative sur le boulon fileté.

7. Dispositif selon la revendication 5 ou 6, **caractérisé en ce que** les éléments de blocage (11) sont agencés aux extrémités libres de la tige (10) subdivisée en deux par la rainure (10a).

8. Dispositif selon la revendication 6 ou 7, **caractérisé en ce que** sur la tige (10) rotative est formé au moins un élément de positionnement (12) qui interagit avec un élément de positionnement correspondant dans la poignée (3).

9. Dispositif selon la revendication 8, **caractérisé en ce que** l'élément de positionnement (12) formé sur la tige (10) est réalisé sous forme de nervure longitudinale, qui peut être insérée dans une rainure longitudinale correspondante dans la poignée (3).

10. Dispositif pour l'amenée de matériau de suture chirurgical à une aiguille, comprenant un corps allongé (1) à l'extrémité distale duquel peut être fixée l'aiguille, et dont l'extrémité proximale est conçue sous forme de poignée (3) munie d'un canal de guidage (4) ouvert pour le matériau de suture à amener, le canal de guidage (4) pouvant être fermé mécaniquement, au moins par secteurs, par l'intermédiaire d'au moins un dispositif de fermeture retenant le matériau de suture (5) dans le canal de guidage (4),
**caractérisé en ce que** ledit au moins un dispositif de fermeture est une agrafe (14) qui peut être fixée sur la poignée (3) et entoure la poignée (3), et qui est réalisée en tant qu'élément de ressort de manière telle que le matériau de suture (5) puisse être enfoncé dans le canal de guidage (4) par le haut, à travers une ouverture (14a) dans l'élément de ressort.

11. Dispositif selon l'une des revendications 1 à 10, **caractérisé en ce qu'**à l'extrémité distale de la poignée (3), dans le corps allongé (1), est formée une cavité de préhension (6) pour la saisie par pincement du matériau de suture (5).

12. Dispositif selon la revendication 11, **caractérisé en ce que** sur le fond de la cavité de préhension (6) est formée une rainure de guidage (6a) pour la réception du matériau de suture (5).
